# EUROPEAN PATENT APPLICATION

(11) **EP 4 491 122 A1**
(43) Date of publication of application: **15.01.2025**
(21) Application number: 23306214.0
(22) Date of filing: 13.07.2023
(51) Int. Cl.: A61B 6/00

(54) **VERSATILE DENTAL ULTRASONIC PROBE**

(71) Applicant: Carestream Dental LLC, Atlanta, GA 30339 (US); TROPHY SAS, 77435 Marne la Vallee Cedex 2 (FR)
(72) Inventor: CAPRI, Arnaud, 77435 MARNE LA VALLEE CEDEX 2 (FR); PETILLO, Julien, 95160 MONTMORENCY (FR); PRON, Michael, 77435 MARNE LA VALLEE CEDEX 2 (FR); ROUDERGUES, David, 77435 MARNE LA VALLEE CEDEX 2 (FR); ALVES MACHADO, Carine, 77435 MARNE LA VALLEE CEDEX 2 (FR)
(74) Representative: Casalonga

(57) **Abstract**

The present invention provides a method of operating an ultrasonic probe comprising a processing unit, a transducer to emit an ultrasound signal and to receive an echoed ultrasound signal, and a communication interface, the ultrasonic probe being configured to be operable in any one of a connected mode and an autonomous mode. Upon determining, in the ultrasonic probe, that the autonomous mode is to be used, data are generated from the ultrasound signals, the generated data are analysed, and a result of the analysis is provided. Upon determining, in the ultrasonic probe, that the connected mode is to be used, data are generated from the ultrasound signals and the generated data are transmitted to another device, the other device providing a result of an analysis of the transmitted data.

## Description

### FIELD OF THE INVENTION

The present invention relates to the field of intraoral measurement methods and devices for the health care industry. Particularly, but not exclusively, the invention relates to a versatile dental ultrasonic probe.

### BACKGROUND OF THE INVENTION

Ultrasound imaging has been adapted for intraoral use in a number of implementations and has been found to have a particular utility for tasks such as measurement of periodontal pocket depth. Conditions such as gingivitis, for example, can be detected by sensing the acoustic response of tissues.

Because of the non-emission of ionizing radiation, ultrasound imaging is inherently safer than ionizing methods and also allows the repeating of an examination if needed. Ultrasound imaging can be used as a substitute for, or a complement to, various types of radiography (cone beam computed tomography or CBCT, panoramic x-ray, or intraoral x-ray imaging), magnetic resonance imaging (MRI), or nuclear medicine.

Ultrasound imaging may use high-frequency sound waves, typically between 1 to 100 MHz. Because high-frequency waves are more attenuated than low-frequency waves for a given distance, high-frequency waves are suitable mainly for imaging superficial structures, e.g. for dermatology, or dental imaging. For example, high-frequency sound waves may preferably have a frequency between 10 to 50 MHz for periodontal pocket investigation. Conversely, low-frequency waves are suitable for imaging the deepest structures of the body. Moreover, image resolution is improved with higher wave frequency.

An ultrasound imaging apparatus generally comprises one or several transducers that act as ultrasound beam emitters and/or ultrasound beam receivers to receive echoes from the emitted signals. In addition, the ultrasound imaging apparatus may comprise various processing and display components used for generating and presenting images from acquired signals. An ultrasound beam emitter generates an ultrasound signal from an electrical signal and conversely, an ultrasound receiver generates electrical pulses from a mechanical ultrasound signal.

Objects in the path of emitted ultrasound signals return a portion of the ultrasound energy back to the transducer which generates electrical signals indicative of the detected objects. Transducers can be of different types among single element transducers or multi-element transducers (annular array, linear array, 2D array, etc.). Furthermore, when a multi-element transducer is used, the emission of ultrasound signals can be delayed in order to enable adaptive focusing. The electronic adaptive focusing makes it possible to increase the resolution depending on the depth of the imaged body part. It also increases transducer sensitivity which improves image contrast.

While the existing ultrasonic probes have proven to be efficient, there is a continuous need for improvement, particularly to maintain their performance and to adapt to the many situations that practitioners may encounter.

### SUMMARY OF THE INVENTION

The present invention has been devised to address one or more of the foregoing concerns.

In this context, there is provided a versatile dental ultrasonic probe configured to operate in multiple modes of operation.

According to an aspect of the invention, there is provided a method of operating an ultrasonic probe comprising a processing unit, a transducer to emit an ultrasound signal and to receive an echoed ultrasound signal, and a communication interface, the ultrasonic probe being configured to be operable in any one of a connected mode and an autonomous mode, the method comprising, in the ultrasonic probe:
upon determining that the autonomous mode is to be used, generating data from the ultrasound signals, analysing the generated data, and providing a result of the analysis , and
upon determining that the connected mode is to be used, generating data from the ultrasound signals and transmitting the generated data to another device, the other device providing a result of an analysis of the transmitted data.

The method according to the invention makes it possible to use the same ultrasonic probe according to different modes depending on the tasks to be performed, on operating conditions, or other considerations, the ultrasonic probe being usable to detect lesions and other problems with patient's gums (e.g., pocket depth measurements, soft tissue inflammation, salivary glands, sub-gingival calculus, tumours, soft tissue diseases such as lychens, oral dermatology diseases, etc.) by imaging the gums and then analysing the acquired data with conventional or Al algorithms.

In an embodiment, the method further comprises determining whether or not a communication connection can be established with a remote device and, upon determining that a communication connection cannot be established with a remote device, obtaining an indicator representing a use of the ultrasonic probe since the last communication connection with a remote device and setting the ultrasonic probe in a fully operational state or in a partially operational state, in the autonomous mode, or in a non-operational state, as a function of the indicator.

The method according to the invention makes it possible to set the ultrasonic probe in a partially operational state or in a non-operational state in particular circumstances, to force a user to connect the ultrasonic probe to a communication network, for example to check the ultrasonic probe, to update software, etc.

In an embodiment, the obtained indicator comprises an item of information representing the date and/or time of the last communication connection with a remote device.

In an embodiment, the obtained indicator comprises an item of information representing a number of available session tokens for using the ultrasonic probe without communication connection.

In an embodiment, the method further comprises updating the obtained indicator.

In an embodiment, the method further comprises filtering the generated data in the autonomous mode and/or in the connected mode and/or comprising analysing the generated data in the connected mode.

In an embodiment, generating data, in the autonomous mode or in the connected mode, comprises reconstructing an image.

In an embodiment, the method further comprises determining whether the ultrasonic probe needs a software update and/or a hardware maintenance.

In an embodiment, the method further comprises synchronizing configuration information of the ultrasonic probe with a remote device.

The method according to the invention makes it possible for the provider of an ultrasonic probe to monitor remotely the use and/or configuration of the ultrasonic probe, to manage software update, and to render the ultrasonic probe unusable in the event of non-compliance.

In an embodiment, the method further comprises receiving and providing, to a user of the ultrasonic probe, acquisition guiding instructions.

According to another aspect of the invention, there is provided an ultrasonic probe comprising a processing unit, a transducer to emit an ultrasound signal and to receive an echoed ultrasound signal, and a communication interface, the ultrasonic probe being configured to be operable in any one of a connected mode and an autonomous mode, wherein, in the autonomous mode, the ultrasonic probe is operable to generate data from the ultrasound signals, to analyse the generated data, and to provide a result of the analysis to a user of the ultrasonic probe, and wherein, in the connected mode, the ultrasonic probe is operable to generate data from the ultrasound signals and to transmit the generated data to another device, the other device receiving the transmitted data, analysing the transmitted data and providing a result of the analysis of the transmitted data.

The ultrasonic probe may be an intraoral ultrasonic probe, for example, an intraoral ultrasonic probe used for measuring periodontal pocket depth. It is possible to use the same ultrasonic probe according to different modes depending on the tasks to be performed, on operating conditions, or other considerations, the ultrasonic probe being usable to detect lesions and other problems with patient's gums (e.g., pocket depth measurements, soft tissue inflammation, salivary glands, sub-gingival calculus, tumours, soft tissue diseases such as lychens, oral dermatology diseases, etc.) by imaging the gums and then analysing the acquired data with conventional or Al algorithms.

In an embodiment, in the autonomous mode, the ultrasonic probe is operational only during a limited time without being communicatively coupled to a remote device or during a limited number of session tokens.

The ultrasonic probe according to the invention may be set in a partially operational state or in a non-operational state in particular circumstances, to force a user to connect the ultrasonic probe to a communication network, for example to check the ultrasonic probe, to update software, etc.

In an embodiment, the transducer is located in a sealed acoustic chamber filled with an internal acoustic coupling material.

According to another aspect of the invention, there is provided an apparatus for measuring tooth pocket depths, the apparatus comprising an ultrasonic probe configured to operate in multiple modes of operation, the ultrasonic probe comprising a processing unit, a transducer to emit an ultrasound signal and to receive an echoed ultrasound signal, and a communication interface, the ultrasonic probe being configured to be operable in any one of a connected mode and an autonomous mode, wherein, in the autonomous mode, the ultrasonic probe is operable to generate data from the ultrasound signals, to analyse the generated data, and to provide a result of the analysis to a user of the ultrasonic probe, and wherein, in the connected mode, the ultrasonic probe is operable to generate data from the ultrasound signals and to transmit the generated data to another device, the other device providing a result of an analysis of the transmitted data.

According to the invention, the same ultrasonic probe may be used according to different modes depending on the tasks to be performed, on operating conditions, or other considerations, the ultrasonic probe being usable to detect lesions and other problems with patient's gums (e.g., pocket depth measurements, soft tissue inflammation, salivary glands, sub-gingival calculus, tumours, soft tissue diseases such as lychens, oral dermatology diseases, etc.) by imaging the gums and then analysing the acquired data with a conventional or AI algorithms.

In an embodiment, in the autonomous mode, the ultrasonic probe is operational only during a limited time without being communicatively coupled to a remote device or during a limited number of session tokens.

According to another aspect of the invention, there is provided a system comprising the ultrasonic probe described above and another device, the other device being configured for receiving the transmitted data, analysing the received data, and providing results of the analysis to a user of the ultrasonic probe.

In an embodiment, the other device is further configured for filtering the received data before the received data are analysed.

In an embodiment, the other device is a remote device, the remote device comprising a dental practice management software, the dental practice management software managing data received from the ultrasonic probe.

In an embodiment, the system further comprises a local device, the local device interfacing the ultrasonic probe and the dental practice management software of the remote device.

According to the invention, the same ultrasonic probe may be used according to different modes depending on the tasks to be performed, on operating conditions, or other considerations, the ultrasonic probe being usable to detect lesions and other problems with patient's gums (e.g., pocket depth measurements, soft tissue inflammation, salivary glands, sub-gingival calculus, tumours, soft tissue diseases such as lychens, oral dermatology diseases, etc.) by imaging the gums and then analysing the acquired data with a conventional or AI algorithms.

The ultrasonic probe may be an intraoral ultrasonic probe, for example an intraoral ultrasonic probe used for measuring periodontal depth pocket.

At least parts of the methods according to the invention may be implemented in a processing unit. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present invention may take the form of a computer program product embodied in any tangible medium of expression having computer usable program code embodied in the medium.

Since the present invention can be implemented in software, the present invention can be embodied as computer readable code for provision to a programmable apparatus on any suitable carrier medium. A tangible carrier medium may comprise a storage medium such as a floppy disk, a CD-ROM, a hard disk drive, a magnetic tape device or a solid state memory device and the like. A transient carrier medium may include a signal such as an electrical signal, an electronic signal, an optical signal, an acoustic signal, a magnetic signal or an electromagnetic signal, e.g. a microwave or RF signal.

### BRIEF DESCRIPTION OF THE DRAWINGS

Embodiments of the invention will now be described, by way of example only, and with reference to the following drawings in which:
**Figure 1** is a perspective view of an example of an ultrasonic intraoral wireless handheld device, according to some embodiments of the invention;
**Figure 2** illustrates schematically an ultrasound system including an ultrasonic probe for measuring pocket depths and for detecting lesions and other problems within a patient's gums;
**Figures 3, 4, and 5** illustrate schematically an example of modules implemented in an ultrasonic probe, in a local device, and in a remote device, respectively;
**Figure 6** illustrates an example of initializing steps of an ultrasonic probe;
**Figure 7** illustrates an example of steps for using an ultrasonic probe in an autonomous mode;
**Figures 8 and 9** illustrate a first and a second examples of steps for using an ultrasonic probe in a connected mode; and
**Figure 10** is a schematic block diagram of a processing device for implementation of one or more embodiments of the invention.

### DETAILED DESCRIPTION OF THE INVENTION

The following is a detailed description of particular embodiments of the invention, reference being made to the drawings in which the same reference numerals identify the same elements of structure in each of the Figures.

In the drawings and text that follow, like components are designated with like reference numerals, and similar descriptions concerning components and an arrangement or interaction of components already described are omitted. Where they are used, the terms "first", "second", and so on, do not necessarily denote any ordinal or priority relation, but may simply be used to more clearly distinguish one element from another, unless specified otherwise.

In the context of the present disclosure, the terms "viewer", "operator", and "user" are considered to be equivalent and refer to the viewing practitioner, technician, or other person who acquires, views, and manipulates an ultrasound image, such as a intraoral image, on a display monitor. An "operator instruction," "user instruction," or "viewer instruction" is obtained from explicit commands entered by the viewer, such as by clicking a button on the ultrasound probe or system hardware or by using a computer mouse or by using a touch screen or a keyboard entry.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

According to some embodiments of the invention, a versatile dental ultrasonic probe is operable in multiple modes for measuring the pocket depths of the gums for one or more of a patient's teeth and, preferably, at least three modes of operation including an autonomous mode, a connected mode, and a connected guided mode. In the autonomous mode, the probe generates data from ultrasound signals, analyses the generated data, and provides a result of the analysis. The autonomous mode may preferably be used only for limited periods of time without communication connection or in a limited number of session tokens (a session token may be defined as one use of the ultrasonic probe on one patient during a predetermined period of time or during the same visit; for the sake of illustration, the period of time may set in the range of 5 minutes to 1hour.). In the connected mode, the probe generates data from ultrasound signals and transmits the generated data to another device, the other device providing a result of an analysis of the transmitted data. In the connected guided mode, the probe operates in communication with another device such as, but not limited to, a laptop computer or smartphone and receives instructions from another device that causes the probe to prompt the probe's user to scan the gums beneath/above a particular tooth or group of teeth. Accordingly, a user is guided step by step for measuring the pocket depths of the gums for one or more of a patient's teeth, the probe generating data from ultrasound signals, for each measure, and transmitting the generated data to a remote device that may analyze the generated data and process the analysis results within a dental practice management software.

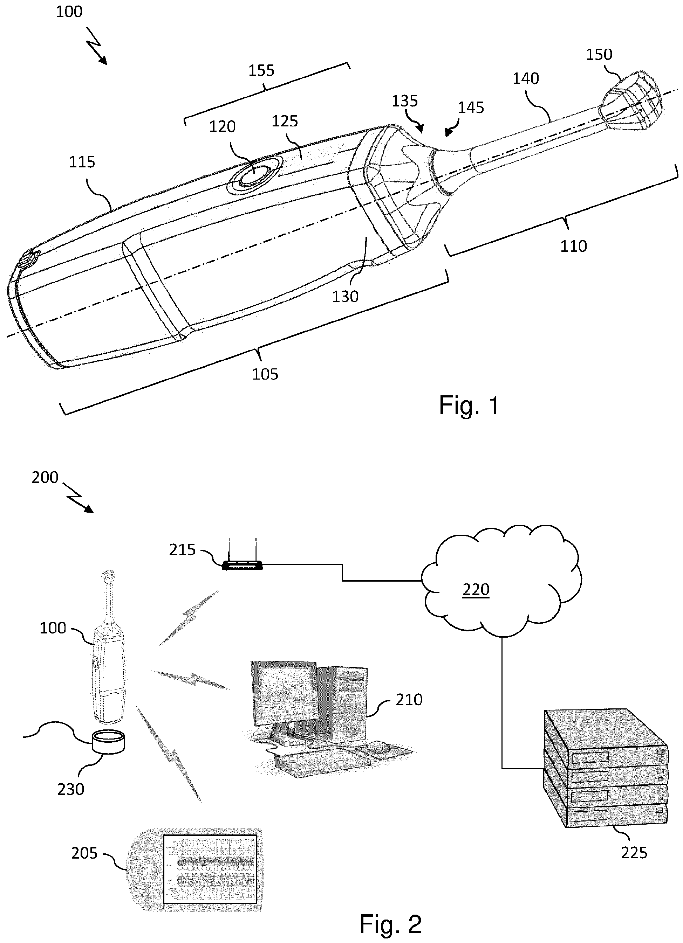

**Figure 1** is a perspective view of an example of an ultrasonic intraoral wireless handheld device 100 (sometimes referred to herein as a handheld probe 100 or probe 100) according to some embodiments of the invention. As illustrated, the ultrasonic intraoral wireless handheld device comprises a probe body 105 and a probe head 110 also called a transducer assembly.

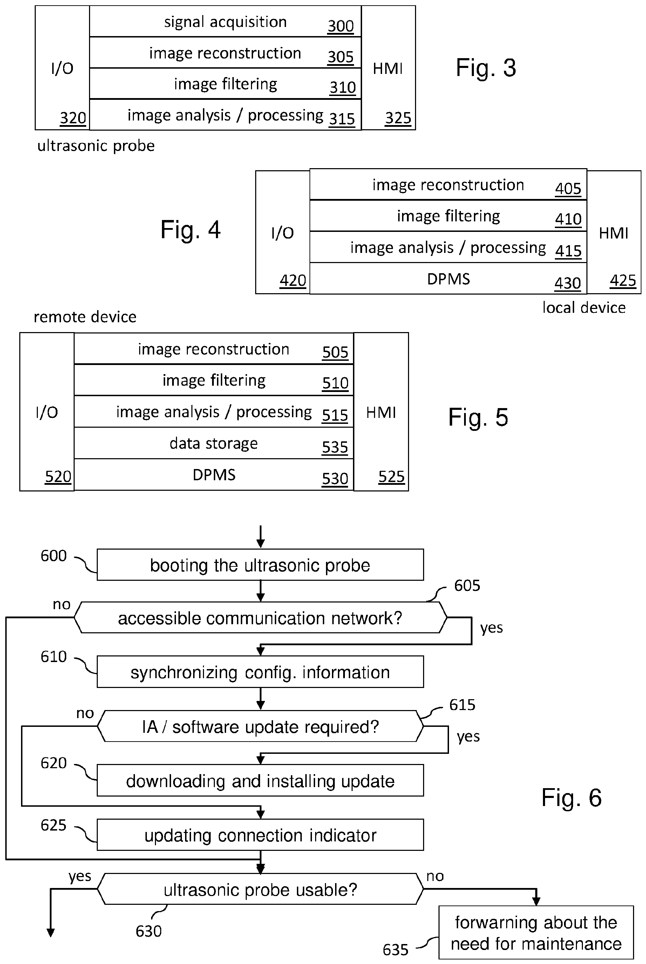

According to this example, probe body 105 comprises a housing 115 that may house a battery and electronic components (not represented), for example a processing device configured to carry out steps as described by reference to Figure 6 to 9. In addition, probe body 105 comprises a user interface 155 making it possible for a user to get information from the probe 100 and to provide instructions to the probe 100. For the sake of illustration, probe body 105 may comprise one or more buttons 120, a display 125, color LEDs (Light Emitting Diodes) 130 (located under the housing and visible through it or located within the housing and visible through an opening in the housing), a buzzer, an inertial measurement unit (not represented) and/or a haptic feedback vibrator (not represented). The inertial measurement unit makes it possible to detect user's actions like taps or double-taps, that may be used to interact with the ultrasonic probe. Collectively, the buttons 120, display 125, LEDs 130, buzzer, inertial measurement unit, and haptic feedback vibrator comprise the user interface 155. It should be understood and appreciated that the user interface 155 may comprise different components or a different combination of components in other embodiments. Using button 120 and short, long, and/or repeated clicks and/or using the inertial measurement unit, the user may browse through menus presented on the display 125 to get information from the probe 100 and/or provide inputs and/or instructions to the probe 100. Probe body 105 also comprises mounting assembly 135 (located under a cover, which may be held in place by an elastic strap, and thus not visible) that makes it possible to mount a probe head 110 to the probe body 105.

As illustrated, probe head 110 comprises a support arm 140 having mounting assembly 145 at one end and a transducer chamber 150 at the other end. Mounting assembly 145 is cooperative with mounting assembly 135 for the probe body 105, so that probe head 110 may be mounted on a probe body 105. It is located under the same cover and thus, it is not visible. Transducer chamber 150 houses one or more transducers that may move (for example, that may oscillate) to emit ultrasound waves toward an object and measure the echo. The transducer is wirelessly connected to electronic components of the probe body 105 or is connected to these electronic components through wires, including, for example and not limitation, coaxial cables. According to some embodiments, the transducer is mounted at one end of a shaft (not represented) that extends longitudinally through the support arm. A cam may be mounted on the other end of the shaft so as to control the movement of the transducer using a motor located in the probe body 105. In addition, the shaft may be hollow to allow the passage of a coaxial wire connecting the transducer to electronic components of the probe body 105.

According to the illustrated example, probe head 110 is a closed probe head 110 having a transducer that is immersed in acoustic chamber 150 filled with an internal acoustic coupling material such as a liquid or gel and sealed.

Ultrasonic probe 100 may be used for clinical applications such as periodontology, implantology, restorative works, and buccal dermatology.

In particular, ultrasonic probe 100 makes it possible to receive signals from which images of the soft tissues of a buccal space may be obtained, these images and/or signals enabling performance of the following tasks:
- detecting morphological abnormalities such as gingival abscess and cancers, and/or
- taking buccal measurements such as measurements of the periodontal pocket depth, of the periodontal gingival margin, of the periodontal attachment level, of the width of an abscess, of anatomical marker distances, etc.
for example, as described in WO2020148406 which is incorporated herein by reference in its entirety.

It is observed that these tasks may be carried out live during the acquisition process (i.e., in real time), or during a post exam operation, once the data set of images and/or raw signal are acquired.

Some of the functions required to carry out these tasks and similar tasks may be grouped in several modules as follows: data acquisition, data analysis and/or processing, data transfer, data storage, notification, and interface with a patient data management system or DPMS (Dental Practice Management Software). These modules may be implemented in the ultrasonic probe 100 and/or in other devices of the system as described with reference to Figure 2. According to some embodiments, some of these modules are implemented in the ultrasonic probe 100 and in other devices of the system, the specific modules to be used are determined according to the circumstances, for example according to the available resources (e.g., available communication bandwidth) or according to a user or practitioner choice. These modules, described by reference to Figures 3, 4, and 5, may be software modules, hardware modules, or modules having a software part and a hardware part. Image analysis and/or processing may comprise identifying textures, patterns, and/or objects, performing measurements, etc.

**Figure 2** illustrates schematically an ultrasonic probe system 200 including an ultrasonic probe, for example ultrasonic probe 100 in Figure 1, for measuring pocket depths and for detecting lesions and other problems within a patient's gums, and for managing patient's data related to such pocket depths, lesions, and other problems.

According to the illustrated example, the system 200 comprises one or more ultrasonic probes 100 and several processing and/or storage devices 205, 210, and 225, that may communicate with each other.

More precisely, ultrasonic probe 100 may transmit data to and/or receive data from a handheld device 205 such as a smartphone, tablet, laptop, and/or a workstation (or personal computer) 210. Handheld device 205 may be accessible by a user or practitioner when manipulating ultrasonic probe 100, for example to display images obtained from the ultrasonic probe, measurements, and/or results of analysis, while workstation 210 may be accessible at the practitioner's desk and used, for example, to handle and manage patient's data. The data sent by ultrasonic probe 100 may be raw data obtained from the transducer of the ultrasonic probe 100, processed data, and/or results of analysis carried out by the ultrasonic probe 100 on such data. The data received by ultrasonic probe 100 may be input and/or instructions for acquiring data and/or results of analysis performed on a remote computing device. These data may be transmitted or received wirelessly, for example using standard communication protocols such as Bluetooth and/or WiFi (Bluetooth and WiFi are trademarks), and/or via a wired connection, for example using a USB (Universal Serial Bus) connection.

Still according to the illustrated example, ultrasonic probe 100 may transmit data to and/or receive data from communication box 215 that may be, for example, a router (e.g., an Ethernet / WiFi router), connected to communication network 220, for example Internet, to which are connected servers 225, for example storage servers and/or data processing servers. According to some embodiments, servers 225 form a cloud.

It is observed that ultrasonic probe 100 may transmit data to and/or receive data from handheld device 205, workstation 210, and/or communication box 215. For example, ultrasonic probe 100 may transmit data to and/or receive data from handheld device 205 so that the user or practitioner may monitor his/her operations in real time on a large screen, to and/or from workstation 210 to process acquired data using a computing device having more resources than handheld device 205, and to communication box 215 to store acquired data in a remote storage server.

The Table in the Appendix provides an example of several possible configurations based on an ultrasonic probe, a workstation, and a cloud (including remote servers and/or data storage devices).

According to the first example of configuration (config. 1), data acquisition, image reconstruction, image analysis, and interactions with the user or practitioner are carried out in the ultrasonic probe 100. Optionally, images and results may be stored in the ultrasonic probe 100.

According to the second example of configuration (config. 2), data acquisition, image reconstruction, image analysis, and interactions with the user or practitioner are carried out in the ultrasonic probe 100. Images and results are stored in the cloud and patient's data are managed in both the workstation and the cloud.

According to the third example of configuration (config. 3), data acquisition, image reconstruction, and image analysis are carried out in the ultrasonic probe. Interactions with the user or practitioner are carried out in the workstation. Images and results are stored in the cloud, and patient's data are managed in both the workstation and the cloud.

The fourth example of configuration (config. 4) is similar to the third example, except that image analysis is carried out in the workstation. Likewise, the fifth example of configuration (config. 5) is similar to the fourth example, except that image reconstruction is carried out in the workstation.

Finally, according to the sixth example of configuration (config. 6), data acquisition, image reconstruction, and image analysis are carried out in the ultrasonic probe. Interactions with the practitioner are carried out in the cloud, images and results are stored in the cloud, and patient data is managed in the cloud.

Turning back to Figure 2, the ultrasound system 200 includes a charging stand 230 that is provided to charge the battery of ultrasonic probe 100. Charging stand 230 may also include communication hardware and software therein and be connected to a communication network, for example to communication network 220 through a wired or wireless connection (not represented), making it possible to upload data stored in ultrasonic probe 100 to local devices or remote servers and/or to download data to ultrasonic probe 100, for example application updates.

It is observed here that the data obtained or generated by the ultrasonic probe 100 are preferably automatically stored in relation with a DPMS, to be used later by a practitioner. This can be done in real-time if the ultrasonic probe 100 is communicatively coupled to a remote device or on a delayed basis in the absence of a communication link.

**Figure 3** illustrates schematically an example of modules implemented in an ultrasonic probe, for example ultrasonic probe 100 in Figure 1.

According to the illustrated example, ultrasonic probe 100 comprises a signal acquisition module 300, an image reconstruction module 305, an image filtering module 310, an image analysis and/or processing module 315, a communication module 320, and a human-machine interface module 325. Some of these modules may be optional, such as the filtering module.

Acquisition module 300 is in charge of acquiring a raw ultrasonic signal (i.e. radio frequency signal) representing the soft tissues to be imaged, within the ultrasonic probe. To that end, electrical energy is transformed into mechanical waves within the transducer (emission), that is directed towards the tissues to be imaged. These mechanical waves are reflected back to the transducer that converts the reflected mechanical waves in an electrical signal (reception). In turn, these electrical signals are sampled in a temporal way to provide raw data representing the tissues to be imaged.

Once these raw data have been obtained, they may be transferred to image reconstruction module 305. These raw data may also be transferred to communication module 320, so that they can be transmitted to a remote device, for example to be stored in a remote storage server and/or to be processed in an image reconstruction module of a local or remote device, for example image reconstruction module 405 in Figure 4 or image reconstruction module 505 in Figure 5. The image reconstruction module processes the raw data to generate images (according to the sectorial acquisition relating to the mechanical swap of the transducer and based on post treatment of the raw data, histogram modifications, geometrical reconstructions, etc.) of the soft tissues to be images. According to some embodiments, reconstruction of images from raw data is based on known algorithms.

Once an image has been reconstructed, it may be transferred to image filtering module 310. The reconstructed image may also be transferred to communication module 320, so that it can be transmitted to a remote device, for example to be stored in a remote storage server and/or to be filtered in an image filtering module of a remote device, for example image filtering module 410 in Figure 4 or image filtering module 510 in Figure 5. The image filtering module processes the reconstructed images. Such a processing may comprise applying one or more image filters, subsampling, etc. The processes applied on the reconstructed images may be based on one or more standard algorithms.

Once an image has been reconstructed and possibly filtered, it may be transferred to image analysis and/or processing module 315. The reconstructed and filtered image may also be transferred to communication module 320, so that it can be transmitted to a remote device, for example to be stored in a remote storage server and/or to be analyzed in an image analysis module of a remote device, for example image analysis and/or processing module 415 in Figure 4 or image analysis and/or processing module 515 in Figure 5. The image analysis module processes the reconstructed and filtered images. Such a processing may comprise segmenting the image, identifying specific features, carrying out measurements, etc. It is observed that the processed applied on the reconstructed images may be based on one or more standard algorithms and/or on one or more artificial intelligence based algorithms. The analysis module may also comprise a step of determining whether the reconstructed and filtered images may be validly used.

Results of the image analysis may be transferred to communication module 320, so that they can be transmitted to a remote device, for example to be stored in a remote storage server and/or to be used in a patient's data management system. Results of the image analysis may also be transferred to a human-machine interface module, for example human-machine interface module 325, to be shown to a practitioner. Likewise, human-machine interface module 325 may display a reconstructed and filtered image. The items of information provided to a practitioner through human-machine interface module 325 may be items of information generated within the ultrasonic probe or received through communication module 320.

**Figure 4** illustrates schematically an example of modules implemented in a handheld device or in a local workstation, for example in handheld device 205 or in workstation 210 in Figure 2, respectively.

According to the illustrated example, the handheld device or local workstation comprises an image reconstruction module 405, an image filtering module 410, an image analysis and/or processing module 415, a communication module 420, a human-machine interface module 425, and a DPMS module 430 (or a module interfacing with a DPMS module of a remote device, acting as a relay between the ultrasonic probe and the remote device).

Image reconstruction module 405 is similar to image reconstruction module 305 described in reference to Figure 3, except that it may reconstruct an image from raw data received through communication module 420, for example raw data received from ultrasonic probe 100.

Likewise, image filtering module 410 is similar to image filtering module 310 described in reference to Figure 3, except that it may filter an image reconstructed by image reconstruction module 405 or an image reconstructed by an image reconstruction module of another device, received through communication module 420, for example an image reconstructed by image reconstruction module 305.

Image analysis and/or processing module 415 is also similar to image analysis and/or processing module 315 described in reference to Figure 3, except that it may analyze an image reconstructed by image reconstruction module 405, possibly filtered by image filtering module 410, or an image reconstructed by an image reconstruction module of another device, received through communication module 420, for example an image reconstructed by image reconstruction module 305 or a reconstructed image filtered by image filtering module 310.

DPMS 430 may manage patient's data received through communication module 420 or resulting from image reconstruction module 405, image filtering module 410, and/or image analysis and/or processing module 415. DPMS may also provide items of information to a practitioner through human-machine interface module 425 or through a human-machine interface module of another device by sending these items of information to the other device through communication module 420.

**Figure 5** illustrates schematically an example of modules implemented in one or more remote servers, for example servers 225 in Figure 2, forming a cloud.

According to the illustrated example, the remote servers comprise an image reconstruction module 505, an image filtering module 510, an image analysis and/or processing module 515, a communication module 520, a human-machine interface module 525, a patient's data management module 530, and a data storage module 535.

Image reconstruction module 505 is similar to image reconstruction module 305 described in reference to Figure 3, except that it may reconstruct an image from raw data received through communication module 520, for example raw data received from ultrasonic probe 100.

Likewise, image filtering module 510 is similar to image filtering module 310 described in reference to Figure 3, except that it may filter an image reconstructed by image reconstruction module 505 or an image reconstructed by an image reconstruction module of another device, and possibly filtered, received through communication module 520, for example an image reconstructed by image reconstruction module 305, possibly filtered by image filtering module 310.

Image analysis and/or processing module 515 is also similar to image analysis and/or processing module 315 described in reference to Figure 3, except that it may analyze an image reconstructed by image reconstruction module 505, possible filtered by filtering module 510, or an image reconstructed by an image reconstruction module of another device, possibly filtered, received through communication module 520, for example an image reconstructed by image reconstruction module 305, possibly filtered by filtering module 310.

DPMS 530 is similar to DPMS 430 described in reference to Figure 4 and may manage patient's data received through communication module 520 or resulting from image reconstruction module 505, image filtering module 510, and/or image analysis and/or processing module 515. DPMS may also provide items of information to a practitioner through human-machine interface module 525 or through a human-machine interface module of another device by sending these items of information to the other device through communication module 520.

Data storage module 535 makes it possible to store items of information received through communication module 520 or resulting from image reconstruction module 505, image filtering module 510, and/or image analysis and/or processing module 515, such as reconstructed images, filtered images, and/or results of analysis of reconstructed images, possible filtered. Data stored by data storage module 535 are preferably stored in shared storage servers, in relation to a patient's data management module, so as to be accessible from several devices.

It is observed here that data obtained from module 300 may be addressed to any of modules 305, 405, and 505 to reconstruct corresponding images. In turn, images reconstructed in any of modules 305, 405, and 505 may be filtered in any of modules 310, 410, and 510, and images reconstructed in any of modules 305, 405, and 505 or images filtered in any of modules 310, 410, and 510 may be analyzed or processed in any of modules 315, 415, and 515. The results of the analysis or processing may be provided to a user via the ultrasonic probe and/or one or several local devices.

Selecting one configuration, for example one of the configurations given in the Appendix, that is to say determining which modules of which devices are to be used, may be based on the available resources, for example the computing resources of the devices in view of the tasks to be done and the communication resources.

For the sake of illustration, if the communication bandwidth is limited, it may be decided to use as much as possible the computing resources of the ultrasonic probe to avoid transferring or communicating a huge amount of raw data. On the contrary, if the communication bandwidth makes it possible to transfer raw data, it may be decided to use the computing resources of a local workstation or of remote servers which can carry out much more complex operations while preserving power of the ultrasonic probe. Naturally, hybrid approaches may be used, for example by reconstructing images within the ultrasonic probe and analyzing the reconstructed images in a local workstation or in remote servers.

In order to address many situations, the ultrasonic probe may be used in different modes, for example in an autonomous mode or in one of a plurality of connected modes.

**Figure 6** illustrates an example of initializing method steps of an ultrasonic probe, for example to initialize ultrasonic probe 100 when powered up.

After booting the ultrasonic probe (step 600), a test is carried out to determine whether communication with a communication network or a remote device is available (step 605). Such a communication connection may be based on the Bluetooth, WiFi protocol, and/or any other similar or different communication protocol. According to some embodiments, a connection with a communication network is requested when the ultrasonic probe is powered up for the first time. If a connection with a communication network or a remote device is available, a communication connection is established, for example with a server of the ultrasonic probe provider (e.g., the probe manufacturer, seller, or lessor). Such a communication connection may be established through a communication box (e.g., communication box 215 in Figure 2). For example, the communication network may be a broadband or local area communication network (including, without limitation, the Internet).

When the communication connection is established, characteristics of the ultrasonic probe (e.g., configuration information) may be uploaded (step 610), for example to a server of the ultrasonic probe provider so that the provider may be aware of these characteristics. For the sake of illustration, such characteristics may comprise a serial number of the probe body, a serial number of the probe head, a model number of the probe head and/or probe body, a version of the embedded software, etc. It is noted that information like a model number could be used by a provider to determine how the probe transmits data, performs certain operations, etc. Likewise, using the serial numbers, the provider may obtain items of information regarding the ultrasonic probe such as the manufacturing date of the different members, their origin, etc. The provider may also verify that these parts have not been stolen and may check the use of the ultrasonic probe (e.g., total operating time, number of measures, etc.). In addition, the provider may also update its databases, for example to store an indication of a change of some parts of the probe, such as the probe head. In such a case, the provider may store the date of change (the date of the first use is preferably stored within the probe and communicated to the provider). It is also observed that the provider may retrieve some information from other information, for example a model number may be retrieved from a serial number.

Conversely, or as a complement, when the communication connection is established, settings of the ultrasonic probe (e.g., configuration information) may be downloaded, for example from a server of the ultrasonic probe provider so as to configure the ultrasonic probe.

Next, it may be determined whether embedded Al models and/or software need to be updated (step 615). In such a case, the corresponding data and/or files are downloaded and installed (step 620). Determining whether embedded Al models and/or software need to be updated may be carried out by the ultrasonic probe in view of information received from the provider or may be carried out by a server of the provider which informs the probe of the need for updating embedded Al models and/or software.

After having updated embedded Al models and/or software or if there is no need for updating embedded Al models and/or software, a connection indicator is updated (step 625). If the connection indicator comprises the date and time of the last connection, it is updated to the current date and time. In addition or alternately, if the connection indicator comprises a number of session tokens in which the ultrasonic probe may be used without access to a communication network, it is updated to the maximum number of session tokens in which the ultrasonic probe may be used without access to a communication network. Such a maximum number of session tokens may be a predetermined value stored in the probe or may be received from the provider.

Next, a test is carried out to determine whether the ultrasonic probe can be used (step 630). This can be done in the ultrasonic probe or in a server of the provider, for example, by checking that none of the members of the probe is out of date. If the ultrasonic probe cannot be used, for example because it requires maintenance, the user or practitioner is advised that the ultrasonic probe cannot be used and that maintenance operations are necessary to use the ultrasonic probe (step 635). Otherwise, the ultrasonic probe may possibly be used as described with reference to Figures, 7, 8, and 9.

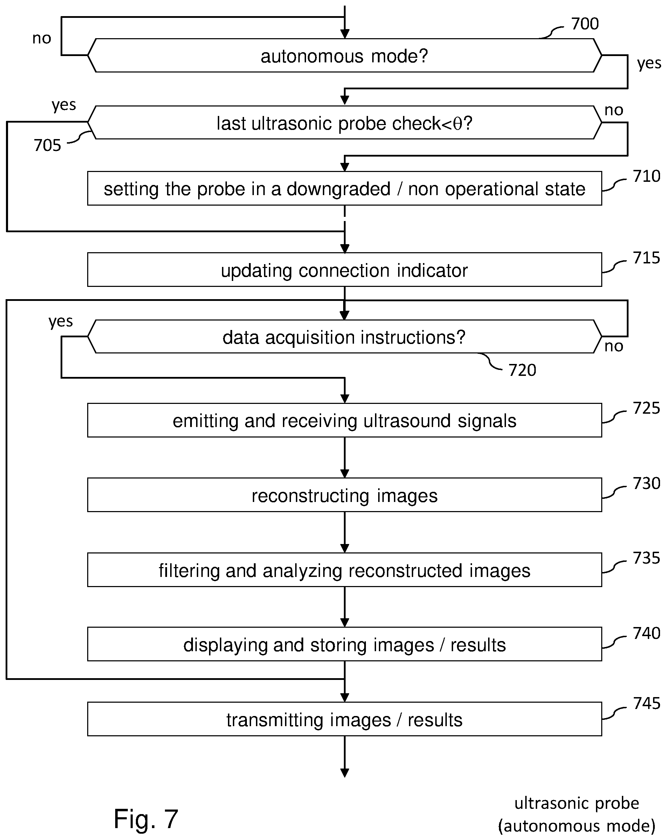

**Figure 7** illustrates an example of method steps for using an ultrasonic probe in an autonomous mode.

It is observed here that while the autonomous mode enables a user or practitioner to use an ultrasonic probe seemingly without any interaction with any other device such as a handheld device, a workstation, or a remote server, a direct or indirect communication connection with a communication network is required from time to time, in particular to check the ultrasonic probe in order to verify that it is operable, for the probe provider to monitor the probe, or for business reasons (e.g., in the case where the cost of a probe depends on its use). In particular, it may be required to verify some parts of the probe, for example the probe head, and/or the version of the embedded software. For the sake of illustration, since the transducer of a closed probe head is generally immersed in a sealed acoustic chamber filled with an internal acoustic coupling material such as a liquid or gel and since the internal acoustic coupling material may present some wear age issues, the probe head should be replaced from time to time.

Checking whether an ultrasonic probe is operable may consist in checking some items of information of the probe such as the version of the software, the manufacturing date of some parts, etc. These items of information may be stored in a database managed by the ultrasonic probe provider, for example in relation to a serial number of the ultrasonic probe.

As illustrated in Figure 7, a first method step is directed to determining whether the ultrasonic probe is to be used according to the autonomous mode (step 700), that may follow step 630 in Figure 6. Operation of the ultrasonic probe in the autonomous mode may result from a choice of the user or practitioner (this mode may be selected from and received via the interface of the probe, e.g., interface 155 in Figure 1) and/or may result from operating conditions, for example if there is no connection with a communication network or another device or if the connection does not make it possible to use reliably another mode.

If the autonomous mode is to be used, a test is carried out to determine whether the ultrasonic probe can be used in a fully operational state (in which all the functions of the autonomous mode may be used), in a partially operational state (in which some of the functions of the autonomous mode cannot be used), or cannot be used, in view of the last connection to a communication network, i.e., depending on whether the time elapsed since the last communication connection is below a given threshold θ (step 705). For example, in the autonomous mode, the probe may be fully operational if the time elapsed since the last communication connection is below a first given threshold θ₁, may be partially operational if the time elapsed since the last communication connection is comprised between the first threshold θ₁ and a second threshold θ₂, and may not be usable if the time elapsed since the last communication connection is above the threshold θ₂ (the second threshold being higher than the first). According to some embodiments, the time elapsed since the last connection is based on a connection indicator, as described by reference to 625 in Figure 6. If the connection indicator comprises the date and time of the last connection, the difference between the date and time of the last connection and the current date and time is computed and compared to a maximum time period allowed. In addition or alternatively, if the connection indicator comprises a number of session tokens, it is determined whether this number of session tokens is equal to or greater than one. If the difference between the date and time of the last connection and the current date and time is greater than the maximum time period allowed and/or if the number of session tokens is smaller than one, the ultrasonic probe is set in a downgraded state (i.e., it is partially operational) or in a non-operational state (step 710). For the sake of illustration, display of measurements may be provided only on the interface of the ultrasonic probe (and not on an interface of a local device) if the difference between the date and time of the last connection and the current date and time is greater than the maximum time period allowed and/or if the number of session tokens is smaller than one. In such a case, the user of the ultrasonic probe is preferably forewarned of the downgraded or non-operational state and asked to connect the ultrasonic probe to a communication network.

Next, if the ultrasonic probe may be used (i.e., the ultrasonic probe is fully or partially operational), the number of session tokens (if the connection indicator comprises a number of session tokens) is decremented by one (step 715).

Next, it is determined whether data acquisition instructions are received (step 720), for example whether a user or practitioner clicks on an acquisition button of the ultrasonic probe.

If data acquisition instructions are received, ultrasound signals are emitted and the echoed signals are measured (step 725). The measured signals are sampled to generate raw data that may be used to reconstruct images (step 730). Next, the reconstructed images may be filtered and analyzed and/or processed (step 735), for example to identify particular patterns, to carry out some measurements, etc. As described above, such an analysis may use conventional algorithms and/or Al based algorithms, it being observed that according to some embodiments, training of the Al algorithms is carried out in a workstation or in one or more servers and the resulting Al model is downloaded in the ultrasonic probe to be used.

The results are then provided to the user or practitioner (step 740), for example displayed on a display, as a code using color LEDs, and/or by means of a sound signal and/or an haptic signal. Optionally, the results and/or the reconstructed images may be stored to be transferred to other devices (step 745), for example to a workstation and/or remote servers. Transmission of the results and/or reconstructed images may be done seamlessly for the user or practitioner or may be done upon requests, for example when the user or practitioner is aware of a communication connection between the ultrasonic probe and another device.

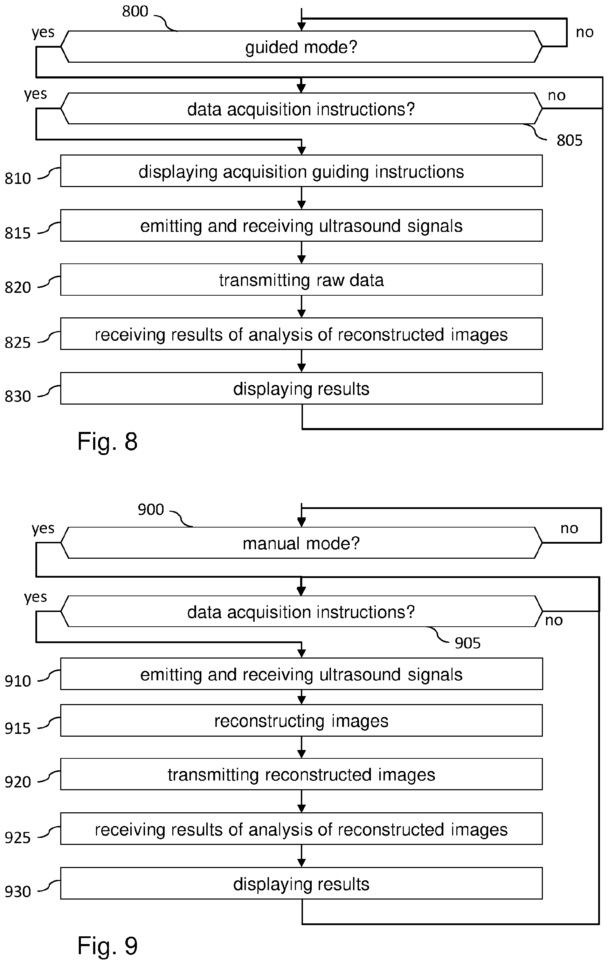

**Figure 8** illustrates an example of steps for using an ultrasonic probe in a connected and guided mode.

According to the connected and guided mode, an ultrasonic probe may receive data from another device, for example guiding instructions to carry out some measurements and transmit data to another device, for example transmit raw data or reconstructed images. For the sake of illustration, upon a request of a practitioner, a DPMS of a remote device may instruct an intermediate module of a local device to guide a user to acquire data with the ultrasonic probe. More precisely, the DPMS may provide general instructions, such as instructions for examining all the periodontal pocket of a patient, to the intermediate module that, in turn, guides the user for measuring the depth of each periodontal pocket.

As illustrated in Figure 8, a first step for using an ultrasonic probe in a connected and guided mode is directed to determining whether the ultrasonic probe is to be used according to the guided mode (step 800). It may follow step 630 in Figure 6, upon determining that a communication network may be accessed.

If the guided mode is to be used, it is determined whether data acquisition instructions are received (step 805), for example whether a user or practitioner clicks on an acquisition button of the ultrasonic probe or whether acquisition instructions are received from another device.

According to the example illustrated in Figure 8, if data acquisition instructions are received, acquisition guiding instructions are provided to the user or practitioner (step 810), for example displayed on a display of the ultrasonic probe. According to some other embodiments, such acquisition guiding instructions are displayed on a display of a handheld device (e.g., a smartphone, a tablet, or a laptop).

For the sake of illustration, such acquisition guiding instructions may provide dynamically the locations of the measures to be done, the probe speed, etc.

Next, ultrasound signals are emitted and the echoed signals are measured (step 815). The measured signals are sampled to generate raw data that may be used to reconstruct images. To that end and according to the illustrated example, the raw data are transmitted to another device (step 820), for example to workstation 210 or servers 225 in Figure 2, wherein the raw data are used to reconstruct images. The reconstructed images are then analyzed and/or processed, in the same device or in another, to identify patterns, to carry out measurements, etc., using conventional algorithms and/or Al based algorithms, as described above.

In response to transmitting the raw data, the ultrasonic probe receives results of the analysis of the images reconstructed from the raw data (step 825). These results are then provided to the user or practitioner (step 740), for example displayed on a display of the ultrasonic probe and/or via a light signal, a sound signal, and/or an haptic signal. Such results are also preferably transmitted to the DPMS (to be stored in relation with the corresponding patient data)

According to the connected and guided mode, multiple user procedures guided by wizard may be displayed on a terminal interface (e.g., the interface of a handheld device) and/or on a display of the ultrasonic probe and/or of a handheld device.

**Figure 9** illustrates an example of method steps for using an ultrasonic probe in a connected and manual mode.

As illustrated in Figure 9, a first step for using an ultrasonic probe in a manual connected mode is directed to determining whether the ultrasonic probe is to be used according to the manual mode (step 900). It may follow step 630 in Figure 6, upon determining that a communication network may be accessed.

Next, it is determined whether data acquisition instructions are received (step 905), for example whether a user or practitioner clicks on an acquisition button of the ultrasonic probe.

According to the example illustrated in Figure 9, if data acquisition instructions are received, ultrasound signals are emitted and the echoed signals are measured (step 910). The measured signals are sampled to generate raw data that may be used to reconstruct images (step 915). Next, according to the illustrated example, the reconstructed images are transmitted to another device (step 920), for example to workstation 210 or servers 225 in Figure 2, wherein the reconstructed images are possibly filtered and then analyzed and/or processed, to identify patterns, to carry out measurements, etc., using conventional algorithms and/or AI based algorithms, as described above.

In response to transmitting the reconstructed images, the ultrasonic probe receives results of the analysis and/or processing of the reconstructed images (step 925835). These results are then provided to the user or practitioner (step 930), for example displayed on a display of the ultrasonic probe and/or of a handheld device.

It is observed that according to some embodiments, the manual mode may switch to the autonomous mode and then back to the manual mode, for example when the communication connection is unstable.

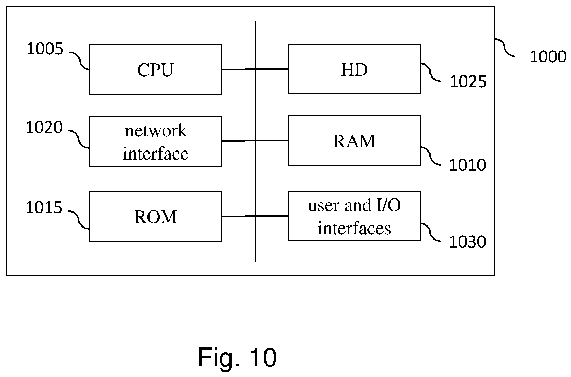

**Figure 10** is a schematic block diagram of a processing device for implementation of one or more embodiments of the invention, in particular for checking and/or for using an ultrasonic probe, as described by reference to Figures 6 to 9.

Computing device 1000 comprises a communication bus connected to:
- a central processing unit 1005, such as a microprocessor, denoted CPU;
- a random access memory 1010, denoted RAM, for storing the executable code of the method of embodiments of the invention as well as the registers adapted to record variables and parameters necessary for implementing a method for training an automatic tooth charting system according to embodiments of the invention, the memory capacity of which can be expanded by an optional RAM connected to an expansion port for example;
- a read-only memory 1015, denoted ROM, for storing computer programs for implementing embodiments of the invention;
- a user interface and/or an input/output interface 1030 which can be used for receiving inputs from a user, displaying information to a user, and/or receiving/sending data from/to external devices; and
- a network interface 1020 typically connected to a communication network over which digital data can be transmitted or received for receiving/sending data from/to remote devices. The network interface 1020 can be a single network interface, or composed of a set of different network interfaces (for instance wired and wireless interfaces, or different kinds of wired or wireless interfaces). Data packets are written to the network interface for transmission or are read from the network interface for reception under the control of the software application running in the CPU 1005.

Optionally, the communication bus of computing device 1000 may be connected to a hard disk 1025 denoted HD used as a mass storage device.

The executable code may be stored either in read-only memory 1015, on hard disk 1025 or on a removable digital medium such as for example a disk. According to a variant, the executable code of the programs can be received by means of a communication network, via the network interface 1020, in order to be stored in one of the storage means of the computing device 1000, such as hard disk 1025, before being executed.

Central processing unit 1005 is adapted to control and direct the execution of the instructions or portions of software code of the program or programs according to embodiments of the invention, the instructions being stored in one of the aforementioned storage means. After powering on, CPU 1005 is capable of executing instructions from main RAM memory 1010 relating to a software application after those instructions have been loaded from ROM 1015 or from hard-disk 1025 for example. Such a software application, when executed by CPU 1005, causes the steps of the methods herein disclosed to be performed.

Any step of the method herein disclosed may be implemented in software by execution of a set of instructions or program by a programmable computing machine, such as a PC ("Personal Computer"), a laptop computer, a tablet, a smartphone, a DSP ("Digital Signal Processor") or a microcontroller; or else implemented in hardware by a machine or a dedicated component, such as an FPGA ("Field-Programmable Gate Array") or an ASIC ("Application-Specific Integrated Circuit").

Although the present disclosure has been described herein above with reference to some specific embodiments, the present invention is not limited to these specific embodiments, and modifications will be apparent to a person skilled in the art which lie within the scope of the present invention.

Many further modifications and variations will suggest themselves to those versed in the art upon making reference to the foregoing illustrative embodiments, which are given by way of example only and which are not intended to limit the scope of the invention, that being determined solely by the appended claims. In particular, the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

**APPENDIX**

| | **config. 1** | **config. 2** | **config. 3** | **config. 4** | **config. 5** | **config. 6** |
|---|---|---|---|---|---|---|
| **acquisit.** | probe | probe | probe | probe | probe | probe |
| **reconstr.** | probe | probe | probe | probe | workstation | probe |
| **analysis** | probe | probe | probe | workstation | workstation | probe |
| **HMI** | probe | probe | workstation | workstation | workstation | cloud |
| **storage** | probe (optional) | cloud | cloud | cloud | cloud | cloud |
| **DPMS** | N/A | workstation / cloud | workstation / cloud | workstation / cloud | workstation / cloud | cloud |

## Claims

1. A method of operating an ultrasonic probe (100) comprising a processing unit, a transducer to emit an ultrasound signal and to receive an echoed ultrasound signal, and a communication interface, the ultrasonic probe being configured to be operable in any one of a connected mode and an autonomous mode, the method comprising, in the ultrasonic probe:
upon determining that the autonomous mode is to be used, generating (725, 730) data from the ultrasound signals, analysing (735) the generated data, and providing (740) a result of the analysis , and
upon determining that the connected mode is to be used, generating (815, 910, 915) data from the ultrasound signals and transmitting (820, 920) the generated data to another device, the other device providing a result of an analysis of the transmitted data.

2. The method of claim 1, further comprising determining (605) whether or not a communication connection can be established with a remote device and, upon determining that a communication connection cannot be established with a remote device, obtaining an indicator representing a use of the ultrasonic probe since the last communication connection with a remote device and setting (710) the ultrasonic probe in a fully operational state or in a partially operational state, in the autonomous mode, or in a non-operational state, as a function of the indicator.

3. The method of claim 2, wherein the obtained indicator comprises an item of information representing the date and/or time of the last communication connection with a remote device.

4. The method of claim 2 or claim 3, wherein the obtained indicator comprises an item of information representing a number of available session tokens for using the ultrasonic probe without communication connection.

5. The method of any one of claims 2 to 4, further comprising updating (715) the obtained indicator.

6. The method of any one of claims 1 to 5, further comprising filtering the generated data in the autonomous mode and/or in the connected mode and/or comprising analysing the generated data in the connected mode.

7. The method of any one of claims 1 to 6, wherein generating data, in the autonomous mode or in the connected mode, comprises reconstructing an image.

8. The method of any one of claims 1 to 7, further comprising determining (615, 630) whether the ultrasonic probe needs a software update and/or a hardware maintenance.

9. The method of any one of claims 1 to 7, further comprising synchronizing configuration information of the ultrasonic probe with a remote device.

10. The method of any one of claims 1 to 9, further comprising receiving and providing (810), to a user of the ultrasonic probe, acquisition guiding instructions.

11. A computer program product for a programmable apparatus, the computer program product comprising a sequence of instructions for implementing each of the steps of the method according to any one of claims 1 to 10 when loaded into and executed by the programmable apparatus.

12. An ultrasonic probe (100) comprising a processing unit, a transducer to emit an ultrasound signal and to receive an echoed ultrasound signal, and a communication interface, the ultrasonic probe being configured to be operable in any one of a connected mode and an autonomous mode, wherein, in the autonomous mode, the ultrasonic probe is operable to generate (630, 635) data from the ultrasound signals, to analyse (640) the generated data, and to provide (645) a result of the analysis to a user of the ultrasonic probe, and wherein, in the connected mode, the ultrasonic probe is operable to generate (725, 820, 825) data from the ultrasound signals and to transmit (730, 830) the generated data to another device, the other device providing a result of an analysis of the transmitted data.

13. The ultrasonic probe of claim 12, wherein in the autonomous mode, the ultrasonic probe is operational only during a limited time without being communicatively coupled to a remote device or during a limited number of session tokens.

14. The ultrasonic probe of claim 12 or claim 13, wherein the transducer is located in a sealed acoustic chamber filled with an internal acoustic coupling material.

15. An apparatus for measuring tooth pocket depths, the apparatus comprising an ultrasonic probe configured to operate in multiple modes of operation, the ultrasonic probe comprising a processing unit, a transducer to emit an ultrasound signal and to receive an echoed ultrasound signal, and a communication interface, the ultrasonic probe being configured to be operable in any one of a connected mode and an autonomous mode, wherein, in the autonomous mode, the ultrasonic probe is operable to generate (630, 635) data from the ultrasound signals, to analyse (640) the generated data, and to provide (645) a result of the analysis to a user of the ultrasonic probe, and wherein, in the connected mode, the ultrasonic probe is operable to generate (725, 820, 825) data from the ultrasound signals and to transmit (730, 830) the generated data to another device, the other device providing a result of an analysis of the transmitted data.

16. The apparatus of claim 15, wherein in the autonomous mode, the ultrasonic probe is operational only during a limited time without being communicatively coupled to a remote device or during a limited number of session tokens.

17. A system comprising an ultrasonic probe (100) according to anyone of claims 12 to 14 and another device (205, 210, 225), the other device being configured for receiving the transmitted data, analysing the received data, and providing results of the analysis to a user of the ultrasonic probe.

18. The system of claim 17, wherein the other device is further configured for filtering the received data before the received data are analysed.

19. The system of claim 17 or claim 18, wherein the other device is a remote device, the remote device comprising a dental practice management software, the dental practice management software managing data received from the ultrasonic probe.

20. The system of claim 19, wherein the system further comprises a local device, the local device interfacing the ultrasonic probe and the dental practice management software of the remote device.
